# EUROPEAN PATENT APPLICATION

(11) **EP 1 013 272 A1**
(43) Date of publication of application: **28.06.2000**
(21) Application number: 98204437.2
(22) Date of filing: 23.12.1998
(51) Int. Cl.: A61K 31/375, A61K 31/40, A61K 31/52, A61K 31/16, A61K 31/35, G01N 33/52

(54) **Manipulation of the activity of a nitric oxide radical production pathway for the treatment of diseases associated with the presence of oxygen free radicals**

(71) Applicant: Biomedical Primate Research Centre (BPRC), 2288 GH Rijswijk (NL)
(72) Inventor: van der Meide, Peter H., 2631 HT Nootdorp (NL)
(74) Representative: Ottevangers, Sietse Ulbe

(57) **Abstract**

The invention discloses a novel pathway for the production of nitric oxide radicals. The invention teaches methods and compounds for affecting the activity of said pathway. At least one of the reaction products in said pathway is an oxygen free radical. By altering the activity of the pathway, diseases may be treated.
A method is disclosed for treating an individual with a condition affectible by oxygen free radicals comprising administering to said individual at least one compound capable of changing the activity of a pathway for the generation of nitric oxide radicals.

## Description

### Field of the invention.

The invention lies in the field of the treatment of diseases. More specifically the invention lies in the field of the treatment of diseases associated with a malfunctioning immune system; particularly, malfunctioning immune systems due to a decline in the number of CD4⁺ T cells and/or an imbalance in T-helper 1 (T_{H}1) and T-helper 2 (T_{H}2) CD4⁺ T cells. In addition, the invention relates to manipulating with the activity of a NO radical production pathway in patients for the treatment of disease.

### Background of the invention.

The immune system is one of the more complex systems in the body. It comprises a large number of different cell types displaying a wide variety of different functions. Some cell types are actively combating foreign invaders or antigens while other cell types have a role in the regulation of the immune response to a certain antigen.
CD4⁺ T cells (CD4-cells) have long since been recognised as playing an important role in the regulation of the immune response. CD4⁺ T cells may interact via the T-cell receptor with peptide-MHC complexes on antigen-presenting cells thereby initiating a cascade of events culminating in an effective immune response. CD4⁺ T cells can be divided into subsets based on the kind of cytokines they produce. T_{H}1 cells typically secrete interferon-γ (IFN-γ) and lymphotoxin whereas T_{H}2 cells produce interleukin 4 (IL-4) and interleukin 5 (IL-5). T_{H}1 cells are thought to control cell-mediated immune responses whereas T_{H}2 cells are thought to be important for B-cell responses.

It is now widely accepted that T cell-dependent immune responses can be classified on the basis of preferential activation and proliferation of two distinct subsets of CD4⁺ T-cells termed T_{H}1 and T_{H}2. These subsets can be distinguished from each other by restricted cytokine secretion profiles. The T_{H}1 subset is a high producer of IFN-γ with limited or no production of IL-4, whereas the T_{H}2 phenotype typically shows high level production of both IL-4 and IL-5 with no substantial production of IFN-γ. Both phenotypes can develop from naive CD4⁺ T cells and at present there is much evidence indicating that IL-12 and IFN-γ on the one hand and IL-4 on the other are key stimulatory cytokines in the differentiation process of pluripotent T_{H}0 precursor cells into T_{H}1 or T_{H}2 effector cells, respectively, *in vitro* and *in vivo* (1) . Since IFN-γ inhibits the expansion and function of T_{H}2 effector cells and IL-4 has the opposite effect, the preferential expansion of either IFN-γ producing cells (pc) or IL-4 pc is indicative of whether an immune response mounts into a T_{H}1 or T_{H}2 direction (2). The cytokine environment, however, is not the only factor driving T_{H} lineage differentiation. Genetic background (3), antigen dose (4), route of antigen administration (5, 6), type of antigen presenting cell (APC) (6, 7) and signalling via TCR and accessory molecules on T cells (8, 9) have all shown to play a role in the differentiation process *in vivo* and there may be other factors. The relative contribution of these various factors in polarising commitment to either T_{H}1- or T_{H}2-type of cells in the initial phase of an immune response is largely unknown. Because predominance of one particular subset can determine health or disease, many studies are currently focused on the identification of genetic predisposing factors that play a role in the selective differentiation of T_{H}0 cells into T_{H}1 or T_{H}2 cells. This knowledge may help to design clinical strategies to redirect pathogenic T_{H} responses in man.
The past few years we have investigated factors and/or mechanism(s) that drive T_{H} subset differentiation. As a model system we used Lewis and BN rats. These two common rat strains exhibit a strong genetic predisposition to mount either T_{H}1 (Lewis) or T_{H}2 (BN) type of immune responses *in vivo* (10, 11) . So far, little information is available about factors and/or mechanisms driving T_{H} subset differentiation in rats. On the other hand, there are a number of papers on this subject in mice indicating that susceptibility to either T_{H}1- or T_{H}2-driven infectious or autoimmune diseases is primarily controlled by genes that lie outside the MHC complex (12-14).
At least some macrophages are known to engage T_{H} cells in a long-lasting interaction resulting in sustained signalling via TCR and costimulatory molecules leading to T_{H} cell activation (15). We reasoned that during this interaction, genetic differences in macrophage functioning may determine which T_{H} subset becomes dominant during an immune response *in vivo*. Our data show that macrophages from BN rats produce significantly greater amounts of both O₂⁻· and H₂O₂ than those of Lewis rats. We found that O₂⁻· was responsible for an increase in NO· bioactivity in the extracellular microenvironment which stimulated T_{H}2-type of immune responses in BN rats by promoting apoptosis in T_{H}1 cells.

Activated CD4⁺ T cells have a variable though finite life span and need to be replenished continuously. This is achieved through a cascade of progenitor cells with more or less self-renewal capability with at the top of the cascade the hemopoietic stem cell. Although CD4-cells are replenished continuously, some diseases are characterised by a decline in the number of CD4-cells. A non-limiting example of such a disease is acquired immune deficiency syndrome (AIDS). A limited decline in the number of CD4-cells can be tolerated by an individual without displaying disease. However, when the decline of CD4-cells proceeds below a certain level, patients have problems with mounting effective immune responses to foreign antigens and, as a result, such patients often suffer from opportunistic infections which can eventually lead to the death of these patients.
Not much is known about the reasons underlying the decline of the number of CD4-cells in for instance AIDS. Although it is generally accepted that human immunodeficiency virus (HIV) causes AIDS, it is not known how the virus causes AIDS. Since HIV specifically infects cells carrying the CD4-receptor, it was first believed that the decline in the number of CD4-cells was a result of the death of HIV infected CD4-cells.
However, this notion is no longer very plausible since most of the dying CD4-cells are actually not infected by the virus. Since the decline of CD4-cells is the underlying cause of AIDS, it is of crucial importance to develop methods that can at least slow down this decline. Much of the attention in the development of treatments for the disease have focused on attacking the causative agent (HIV) or the opportunistic infections. Though this has resulted in a steady improvement of the quality of life and the life expectancy of patients, it has not yet resulted in a cure for the disease.

### Summary of the invention

The present invention discloses a novel pathway leading to CD4 T cell death. The present invention teaches methods and compounds to change the activity of this pathway. The methods and compounds of the present invention are useful in the treatment of patients exhibiting a decline in the number CD4⁺ T cells, or exhibiting an imbalance of CD4⁺ T-helper 1 and T-helper 2 cells. The present invention further teaches at least one of the underlying causes for the progressive decline in the number of CD4⁺ T cells in patients infected with HIV. The invention provides methods and compounds that can at least slow down a decline in the number of CD4-cells in patients.

### Brief description of the drawings

Figure 1
   Nitrite levels in splenocyte cultures from saline- and HgCl₂-exposed rats. Splenocytes were obtained at 72 h after the first injection with either NaCl or HgCl₂. Cells were stimulated with Con A in the presence of 2 mM GSH for 48 h at 37°C. The nitrite concentration in the culture supernatant was determined by the colorimetric Griess reaction. One representative experiment out of 3 in quadruplicate is presented.
Figure 2
   Visualisation of spot forming cells by ELISPOT analyses. The picture shows the number of IL-4 pc (per 2 x 10⁵ MNC) and IFN-γ pc (per 10⁴ MNC) in cultures of rat splenocytes with (360 µM) or without NMMA. Splenocytes originated from HgCl₂-exposed BN rats.
Figure 3
   The GSH-mediated decomposition of GSNO as a function of time and GSH concentration. For each experiment GSNO was prepared freshly. The reactions (in 3 ml reaction vials protected from light) were performed in 10 mM potassium phosphate buffer at pH 7.4 (45). In the presented experiments 0.1 mM GSNO was incubated for varying length of time at room temperature in the presence of GSH ranging from 0 to 32 mM. GSNO decomposition was followed by measuring the decrease in absorbance at 335 nm. Three independent experiments were performed with similar results. One representative experiment is presented.
Figure 4
   Nitrite formation as a function of the ratio GSH/GSNO. Synthetically produced GSNO (0.1 mM) was incubated with varying amounts of GSH for 42 h at room temperature in the dark. The amount of nitrite formed during incubation was measured by the colorimetric Griess reaction. One representative experiment out of 3 is presented.
Figure 5
   Inverse relationship between the formation of nitrite and ammonia during the GSH-mediated decomposition of GSNO. GSNO (0.1 mM) was incubated with increasing amounts of GSH for 42 h at room temperature in sealed vials protected from light. The amount of NH₃ was determined by a commercial assay which is based on the reductive amination of 2-oxoglutarate by glutamate dehydrogenase and NADPH. Nitrite levels were measured by the colorimetric Griess reaction. Two independent experiments were performed which produced similar results. One experiment is presented.
Figure 6
   The effect of an exogenous source of superoxide anions on nitrite formation during the GSH-mediated decomposition of GSNO. GSNO (0.1 mM) was incubated with varying amounts of GSH for 42 h at room temperature in sealed vials protected from light. Reaction mixtures received no supplements (control), HY (0.1 mN), XO (5 mU) or both XO and HY. After 42 h of incubation nitrite levels were measured by the colorimetric Griess reaction. In concert with 0.1 mM HY, XO was found to dose-dependently increase nitrite levels. One representative experiment (using 5 mU XO) out of 3 is presented.
Figure 7
   Proposed reaction pathways for the superoxide anion-mediated stimulation of NO^{·}/NO₂⁻ formation during the GSH-triggered breakdown of GSNO. The reactions of the GSH conjugate N-hydroxysulfenamide (GS - NOH - SG) with GSH and the GSH N-hydroxyl radical (GS - N^{·}- OH) with molecular oxygen which produce NH₃ and NO₂⁻, respectively, have thoroughly been studied by Singh et al. (45).

### Detailed description of the invention

A basic aspect of the invention is a novel pathway for the synthesis of nitric oxide radicals (NO·). NO· is a compound with pleiotropic effects. One of the functions of NO· is that it induces death, presumably through apoptosis, of T-helper-1 cells.

We have discovered that said novel pathway for the synthesis of NO· is a cause for the selective death of CD4-T_{H}1 cells in a number of immune-mediated disorders.

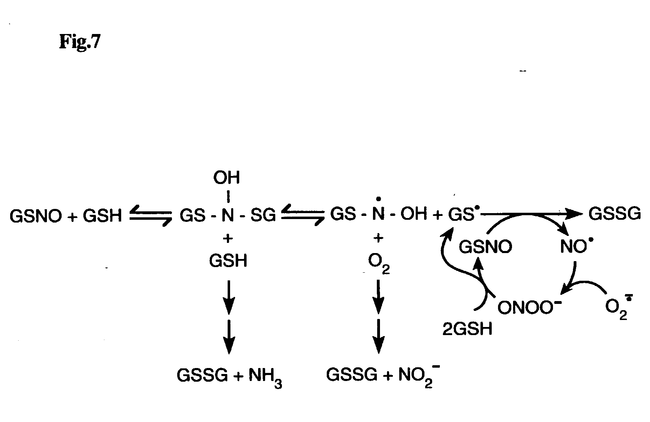

The discovered pathway, designated the Rijswijk-cycle is depicted in figure 7. In this cycle, NO· reacts with O₂⁻· to form peroxynitrite. This peroxynitrite is subsequently converted by thiols, such as glutathione or cysteine into S-nitrosothiols such as S-nitrosoglutathione or S-nitrosocysteine, respectively. These S-nitrosothiols can interact with thiyl radicals to produce NO· radicals and oxidised thiols.
The pathway may be seen as a salvage pathway to rapidly detoxify O₂⁻· radicals. In the process of detoxifying O₂⁻· radicals, the cycle generates new NO· leading to a, per time unit, increased production of NO·. Though the absolute concentration of NO· may not necessarily increases and may even decrease due to the activity of the cycle, the increased production of NO· leads to an increase in the effect of the NO·. The increase in NO· production may be explained by a shift in the equilibrium between GS-NOH-SG and GS-N^{·}-OH + GS· (in figure 7) to the right which would lead to an increased generation of NO· per time unit from available molecules such as GSNO. One of the effects of the increased NO· production is an increase in the NO· mediated death of T_{H}1-cells, presumably through apoptosis, which may become detrimental for an individual, for instance when enhancement of NO· production is sustained for a substantial period of time. In case of a peak production of O₂⁻· radicals, the beneficial effect of removing O₂⁻· radicals from the system may outweigh a potential harmful effect of the increased production per time unit of NO·. However, in case of prolonged production of O₂⁻· radicals, harmful effects of NO· may become apparent.
In the following non-limiting example, we describe one of the ways in which activity of the Rijswijk-cycle may have detrimental effects.

In patients carrying an HIV infection, macrophages become infected with the virus. In at least some of the infected macrophages O₂⁻· radicals are produced. This production may be continuous or in bursts. However, considering the large number of macrophages that can become infected with HIV, the effect of even burst production of O₂⁻· radicals will be a continuing presence of newly generated O₂⁻· radicals.
The produced O₂⁻· radicals will at least partly be scavenged by the Rijswijk cycle. As a consequence NO· radical production per time unit, at least in the vicinity of the infected macrophages, will be increased significantly when compared to the NO· radical production per time unit in the vicinity of macrophages, from healthy uninfected individuals.
Moreover, once macrophages become infected in an HIV infected individual, infected macrophages will be present in that individual for most of the remaining period of life. Thus presenting a continuous source of O₂⁻· radicals and thus a continuous increased NO· radical production per time unit.

One of the functions of macrophages is the presentation of antigens to T-cells. As such, many macrophages in the body, for instance dendritic cells, are closely associated with T-cells, among which CD4⁺ T cells. Both T-helper 1 and T-helper 2 cells may be present in the vicinity of macrophages.
Particularly T-helper 1 cells are sensitive to NO· radical induced cell death, presumably through apoptosis. In HIV-infected individuals, at least some of the HIV-infected macrophages produce O₂⁻· radicals and thus the Rijswijk-cycle will be active in the vicinity of these infected macrophages.
Activity of the Rijswijk cycle will result in an increased production of NO· radicals in the vicinity of infected macrophages, such as dentritic cells, and thus also in the vicinity of the CD4⁺ positive T cells associated with macrophages. Since NO· radicals induce cell death of T-helper 1 cells, at least part of this subset of T-cells may die. As a result of the disappearance of T-helper 1 cells the immune system may favour a T-helper 2 cell controlled type of immune response and/or diminish CD4⁺ T cell count. The activity of the Rijswijk cycle in HIV-infected individuals is proposed to form at least one the causes for the loss of uninfected CD4⁺ T cells and impairment of cellular immune responses, in HIV-infected individuals.
In one of the aspects of the invention are presented methods and compositions for changing the activity of the Rijswijk cycle in HIV-individuals leading to at least a decrease in the decline of CD4⁺ T cells and thus leading at least to an improved capacity of the immune system in these individuals to mount effective immune responses against opportunistic infections.

Manipulating the activity of the Rijswijk-cycle may also have beneficial effects in the treatment of other diseases than AIDS. In diseases where T_{H}2 cells play a prominent disease promoting role, such as for example allergic diseases, that role may be decreased through decreasing the activity of the Rijswijk cycle. Decreasing the activity of the Rijswijk cycle leads to a decrease in the Rijswijk-cycle related kill of T_{H}1 cells leading to at least a decrease in the disease promoting role of T_{H}2 cells.
In other diseases where T_{H}1 cells play a prominent disease promoting role, that role may be decreased through increasing the activity of the Rijswijk cycle. Increasing the activity of the Rijswijk cycle leads to an increase in the Rijswijk cycle related kill of T_{H}1 cells leading to at least a decrease in the disease promoting role of T_{H}1 cells.
The activity of the Rijswijk cycle may be increased through supplying compounds of which the amount in the body in the patients is limiting the activity of the Rijswijk cycle. One such compound that may be supplied is a compound that increases the O₂⁻ radical production by macrophages, preferably of macrophages in the vicinity of T_{H}1 cells.
Another example of a compound which, when supplied to patients, will result in an increase of the Rijswijk cycle is peroxynitrite, or compounds that result in the production of peroxynitrite in the body, preferably in the vicinity of T_{H}1 cells. It is clear that for this purpose also functional analogues of peroxynitrite may be used, for instance but not limited to compounds capable of generating thiyl radicals or compounds that alone or in combination are capable of generating O₂⁻· and/or NO·, preferably in equimolar amounts, such as 3-morpholinosidnonimine (SIN-1). It is also clear that for this purpose derivatives of peroxynitrite, similar in kind of activity though not necessarily similar in the amount of activity, may be used.

In one embodiment of the invention is provided a method for manipulating the immune response in an individual by influencing the activity of the Rijswijk cycle. In a preferred embodiment of the invention the immune response is at least in part manipulated toward a more T_{H}1-type of immune response by decreasing the activity of the Rijswijk cycle. In another preferred embodiment of the invention the immune response is at least in part manipulated toward a more T_{H}2-type of immune response by increasing the activity of the Rijswijk cycle.

A preferred means for changing at least in part, the activity of the Rijswijk cycle, is a means that is directed to molecules, participating in the Rijswijk cycle, of which the concentration in the body may be altered without interfering, in an essential way, with the function of other processes in the body. For instance, removal or reduction of the amount of NO· radicals is only an applicable therapy when such removal is located predominantly in the vicinity of CD4⁺ T cells or macrophages. Removal or reduction of the amount of NO· radicals from the entire body is not preferred considering the pleiotropic effects of NO· radicals, which include favourable effects. These favourable effects should preferably not be interfered with. Conversely, increasing the amount of NO· radicals in the entire body is similarly not desired because of the pleiotropic effects of the compound which include detrimental effects.

A non-limiting example of a molecule of which the concentration in the body may be altered at least in part without affecting in a serious manner, important other processes in the body is peroxynitrite. One of embodiments of the invention therefore encompasses means and methods for the conversion of peroxynitrite into substances that do not participate in the Rijswijk cycle. By decreasing the amount of peroxynitrite capable of participating in the Rijswijk cycle, the NO· radical generating capacity of the cycle is reduced. A favourable aspect of this so-called scavenging of peroxynitrite is that the O₂⁻· scavenging component of the Rijswijk cycle is at least partly, only minimally affected.

In one aspect of the invention are provided methods and compounds for the treatment of an individual with a disease associated with the presence of oxygen free radicals. Said individual may have the disease at the moment of treatment but may also be at risk for developing disease, as long as at the time of treatment oxygen free radicals are present in the patient. Said methods and compounds comprise, but are not limited to, the administration to said individual of at least one type of compound capable of scavenging peroxynitrite.
Compounds that scavenge peroxynitrite may be any compound suitable for use as a pharmaceutical as long as the compound, or derivatives thereof, function to decrease the amount of peroxynitrite available to participate in the Rijswijk cycle.
Compounds that react with peroxynitrite but where the reaction product can participate in the Rijswijk cycle can also be used for the present invention when such reaction products result in a reduced activity of the Rijswijk cycle.
Preferred compounds that may be administered to a patient for the scavenging of peroxynitrite are compounds selected from the groups of porphyrin and purine derivatives such as 5,10,15,20-tetrakis(2,4,6-trimethyl-3,5-disulfonatophenyl)-porphyrinato ironIII(FeTMPS) and uric acid respectively, and compounds such as deferoxamine (desferrioxamine), ascorbate and Trolex. CSH and GSH also react with peroxynitrite however, these compounds have the disadvantage that at least some of the reaction products may take part in the Rijswijk cycle. Deferoxamine, ascorbate and Trolex are also compounds that may react with peroxynitrite and may at least in part inactivate it.
Said compounds may be used separately or two or more compounds may be used simultaneously. In the latter case said simultaneous use may lead to synergistic effects on the activity of the Rijswijk-cycle.
In principle any compound capable of donating an H⁺ is a peroxynitrite scavenger. An acidic environment will promote the scavenging of peroxynitrite. Preferably however, H⁺ donors active at physiological pH are used. Suitable peroxynitrite scavengers may be selected from the group of natural compounds comprising an sulfhydryl group. Suitable peroxynitrite scavengers may be found in compounds which possess reducing capacity.

Peroxynitrite scavengers should be administered to patients in amounts effective in reducing the amount of peroxynitrite available for participating in the Rijswijk cycle. Preferably the scavengers of peroxynitrite are administered in amounts effective for reducing a decline in the number of CD4⁺ T cells of the T-helper 1 subset.

Compounds to affect the activity of the Rijswijk cycle may be administered to individuals through a, for a particular substance, suitable route. For instance, but not limited to uric acid and/or FeTMPS which compounds may be administered for instance subcutaneously in suitable doses.

A purine derivative such as uric acid may be administered subcutaneously, preferentially by a slow-release system to reach effective plasma levels. In one embodiment of the invention uric acid is administered subcutaneously by a slow release system to reach plasma levels ranging from 2 to 20 µg/ml.
Uric acid administration by the oral route can also be envisaged. Doses of 10 to 50 gram suspended in an appropriate buffering solution such as phosphate buffered saline at physiological pH (7.4) may be taken orally, for example 3 times a day, until the end of the treatment. An envisaged end point for treatment is for example when nitrite/nitrate levels decrease to levels normally found in the circulation of healthy individuals. Another non-limiting end point of treatment would be when the number of CD4 cells has improved to the number normally found in the circulation of healthy individuals.
Porphyrin derivatives such as FeTMPS may be administered by similar routes. In one embodiment of the invention porphyrin derivatives such as FeTMPS are administered the an individual at doses ranging from 10 to 100 mg/kg/b.w. provided that said individual does not exhibit obvious adverse side-effects associated with the administration of said derivatives.
Both purine and porphyrin derivatives may be administered separately, in concert with each other or in combination with other peroxynitrite scavengers such as compounds comprising sulphydryl moieties, desferoxamine, ascorbate or Trolex.

In a preferred embodiment of the invention compounds are used for the scavenging of peroxynitrite that change the activity of a nitric oxide radical production pathway, preferably the Rijswijk cycle.
In one aspect of the invention peroxynitrite scavengers are used for the treatment of an individual exhibiting a declining number of CD4⁺ T cells, associated with the presence of oxygen free radicals. In another aspect of the invention a peroxynitrite scavenger is administered to individuals carrying a lentivirus infection, where one of the effects of said administration is a decrease in the decline of CD4⁺ T cells in said individual, associated with the presence of oxygen free radicals. An important example of such a lentivirus is human immunodeficiency virus. In a preferred embodiment of the invention the methods and compounds of the invention are used in a treatment of an individual suffering from lentivirus induced acquired immune deficiency syndrome or for the treatment of an individual at risk of developing such syndrome. The methods and compounds of the invention are also useful for the treatment of animals or humans presenting or at risk of presenting with a disease associated with the presence of oxygen free radicals.
The activity of the Rijswijk cycle in an individual can be monitored through determining the levels of ammonia and/or nitrite (or derivatives or metabolites of both compounds) in, for example, a blood sample taken from an individual. When the Rijswijk cycle is not or only minimally active the ratio of the stable nitrogen metabolites ammonia/nitrite is relatively high. With increasing activity of the Rijswijk cycle the production and the amount of nitrite increases, thereby lowering the ammonia/nitrite ratio. Both the ratio and the amounts of ammonia and nitrite produced, or derivatives thereof, form an indication of the activity of the Rijswijk cycle. For example, activity of the Rijswijk cycle in HIV-patients forms an explanation for the observed elevated levels of nitrite in blood of HIV-patients which correlate with disease activity and CD4⁺ T lymphocyte count.
Levels of ammonia, nitrite or derivatives or metabolites thereof may be determined in samples taken from an individual, preferably in a blood or a urine sample. Both the amount and the ratio between ammonia and nitrite form an indication of the activity of the Rijswijk cycle. Ammonia and nitrite are not only produced in the processes mentioned above, thus the assays for determination of ammonia and/or nitrite may only be used for the determination of the activity of the Rijswijk cycle under conditions were activity of the Rijswijk cycle is expected to be the predominant factor in the production of ammonia and/or nitrite. In one aspect of the invention is therefore provided a method for diagnosing an individual, suspected of having an increased production of oxygen radicals, exhibiting a disease or at risk of exhibiting a disease, comprising detecting in a sample taken from said individual ammonia and/or nitrite, or derivatives or metabolites of said compounds.
In another aspect of the invention is provided a peroxynitrite scavenger or a composition comprising a peroxynitrite scavenger. Preferably said scavenger or said composition comprises at least one of the compounds selected from the groups of porphyrin and purine derivatives such as 5,10,15,20-tetrakis(2,4,6-trimethyl-3,5-disulfonatophenyl)-porphyrinato ironIII(FeTMPS) and uric acid respectively, and compounds such as deferoxamine (desferrioxamine), ascorbate and Trolex.
In yet another aspect of the invention is provided a pharmaceutical composition comprising a peroxynitrite scavenger together with a pharmacologically acceptable carrier. This pharmaceutical composition may be used for the treatment of an individual exhibiting a declining number of CD4⁺ T cells, associated with the presence of oxygen free radicals, said treatment comprising administering to said individual at least one compound capable of scavenging peroxynitrite.

Scavengers of peroxynitrite may also be used for preparing a pharmaceutical composition for the treatment of an individual exhibiting a declining number of CD4⁺ T cells, associated with the presence of oxygen free radicals. Such pharmaceutical compositions may comprise as the scavenger one or more of the compounds selected from the groups of porphyrin and purine derivatives such as 5,10,15,20-tetrakis (2,4,6-trimethyl-3,5-disulfonatophenyl)-porphyrinato ironIII(FeTMPS) and uric acid, respectively and compounds such as deferoxamine (desferrioxamine), ascorbate and Trolex.

In one embodiment the invention provides a method for treating an individual with a condition capable of being influenced by oxygen free radicals and/or nitric oxide free radicals comprising administering to said individual at least one compound capable of changing the activity of a pathway for the generation of nitric oxide radicals. In a preferred embodiment the activity of a pathway for the generation of nitric oxide radicals is changed in the vicinity of oxygen free radicals producing cells.
In another preferred embodiment the activity of a pathway for the generation of nitric oxide radicals is changed in the vicinity of CD4⁺ T helper cells. In yet another preferred embodiment the activity of a pathway for the generation of nitric oxide radicals is changed in the vicinity of cells of the central nervous system.
In a preferred embodiment said pathway is the Rijswijk cycle.
Preferably said individual has a condition associated with a malfunctioning immune system. In a preferred embodiment said compound is capable of decreasing the activity of said pathway. Preferably said compound is a scavenger of peroxynitrite. Preferably said compound is selected from the groups of porphyrin and purine derivatives such as 5,10,15,20-tetrakis(2,4,6-trimethyl-3,5-disulfonatophenyl)-porphyrinato ironIII(FeTMPS) and uric acid, respectively, and comounds such as deferoxamine (desferrioxamine), ascorbate and Trolex and a combination of two or more of said compounds.
In a preferred embodiment the invention provides a method for the treatment of an individual suffering from a condition associated with a declining number of CD4 cells. In a particularly preferred embodiment the invention provides a method for the treatment of an individual suffering from a lentivirus infection, for instance a human immunodeficiency virus infection.
In one embodiment the invention provides a method for the treatment of an individual suffering from an allergic disease.
In another embodiment the invention provides a method for the treatment of an individual with a condition capable of being influenced by the presence of oxygen free radicals and/or nitric oxide free radicals comprising administering to said individual a compound capable of increasing the activity of a pathway for the generation of nitric oxide radicals. In a preferred embodiment the compound is peroxynitrite, a derivative of peroxynitrite, a peroxynitrite generating compound or a functional analogue of peroxynitrite. In a preferred embodiment said compound is used to at least in part decrease a T-helper 1 type immune response in said individual. Preferably said compound is administered in an amount effective for changing the activity of said pathway.
Preferably said compound is administered in an amount effective for changing at least partly the immune response in an individual.
In another embodiment the invention provides a method for diagnosing an individual suffering from a condition associated with an increased production of oxygen radicals, comprising providing a sample from said individual and detecting or measuring or both, ammonia and/or nitrite, or derivatives of said compounds in said sample.

In another embodiment the invention provides a peroxynitrite scavenger for use in a method for treating an individual with a condition capable of being influenced by oxygen free radicals and/or nitric oxide free radicals. In a preferred embodiment said peroxynitrite scavenger is selected from the groups of porphyrin and purine derivatives such as 5,10,15,20-tetrakis(2,4,6-trimethyl-3,5-disulfonatophenyl)-porphyrinato ironIII(FeTMPS) and uric acid, respectively, and compounds such as deferoxamine (desferrioxamine), ascorbate and Trolex.
In another embodiment the invention provides a pharmaceutical composition comprising a peroxynitrite scavenger together with a pharmaceutically acceptable carrier. In a preferred embodiment said pharmaceutical composition is used for the treatment of an individual exhibiting a declining number of T helper 1 cells, associated with the presence of oxygen radicals, said treatment comprising administering to said individual at least one compound capable of scavenging peroxynitrite. In another preferred embodiment said pharmaceutical composition comprises an amount of effective for at least reducing the decline of the number of T helper 1 cells.
In another embodiment the invention provides a method for treating an individual with a condition capable of being influenced by oxygen free radicals and/or nitric oxide free readicals comprising administering to said individual at least one compound capable of changing the activity of a pathway for the generation of nitric oxide radicals in order to prevent at least in part a change in the activity of said pathway. For instance but not limited to a situation where an individual has been infected with human immunodeficiency virus but as yet no oxygen free radicals are produced as a result of the infection. In this case a peroxynitrite scavenger may be administered to said individual to prevent at least in part a change in the activity of the Rijswijk cycle. Thereby, preventing at least in part a decline in the number of CD4⁺ T cells due to the activity of the Rijswijk cycle.

The invention will now be illustrated by the following non-limiting examples, in which the results show that macrophages of normal BN rats produce higher amounts of reactive oxygen intermediates (ROI) than those of Lewis rats. O₂⁻· was found to stimulate the release of NO^{·} from S-nitrosothiols (RSNO), a class of compounds that can be produced by activated macrophages via the intermediacy of thiols and peroxynitrite and have the intrinsic capacity to buffer NO^{·} bioactivity *in vitro* and *in vivo*.
The increase in NO^{·} levels had an impact on T_{H} subset differentiation in the BN spleen. At least under conditions of polyclonal T cell stimulation leading to a certain level of NO^{·} activity, NO^{·} induced killing was more pronounced in T_{H}1 cells than in T_{H}2 cells, thereby favouring T_{H}2-type of immune responses. This selective killing of T_{H}1 cells related to NO^{·} was established by ELISPOT analyses allowing the enumeration of both IFN-γ- and IL-4-producing T cells in rat splenocyte cultures. These analyses revealed that splenocytes from-normal BN rats generated much higher numbers of IL-4 pc and less IFN-γ pc than those from Lewis rats. The relative low ratio of IFN-γ pc/IL-4 pc in BN splenocyte cultures could be enhanced by inhibiting NO^{·} synthesis, suggesting that BN rats exhibit an inherited preference to mount T_{H}2-type of immune responses by a mechanism involving NO^{·} formation.
When we exposed BN rats to low dose HgCl₂, the ratio of the mitogen-induced generation of IFN-γ pc/IL-4 pc in splenocyte cultures was reduced, concurrently with an increase in the production of both reactive oxygen intermediates (ROI) and nitrite. Inhibition of NO^{·} synthesis in these cultures by adding NMMA, augmented the ratio of IFN-γ pc/IL-4 PC, indicating that HgCl₂ administration not only stimulated ROI production but also led to a NO^{·} -mediated polarisation towards a T_{H}2-type of immune response in BN spleen.
A high frequency of IL-4 pc as observed in BN splenocyte cultures may have implications in the context of ROI production. IL-4 is an inhibitor of the IFN-γ-induced synthesis of ROI by macrophages from various species (48, 49), suggesting that the preferred generation of IL-4 pc in BN spleen cell cultures may in fact represent a feed-back mechanism to counterregulate the high level production of ROI by BN macrophages.
It should be noted that exposure of Lewis rats to HgCl₂ also led to an increase in ROI production and a decrease in the ratio of splenic IFN-γ pc/IL-4 pc which is in line with the reported suppressive effect of HgCl₂ on the T_{H}1-mediated autoimmune disease EAE in these animals (50, 51).

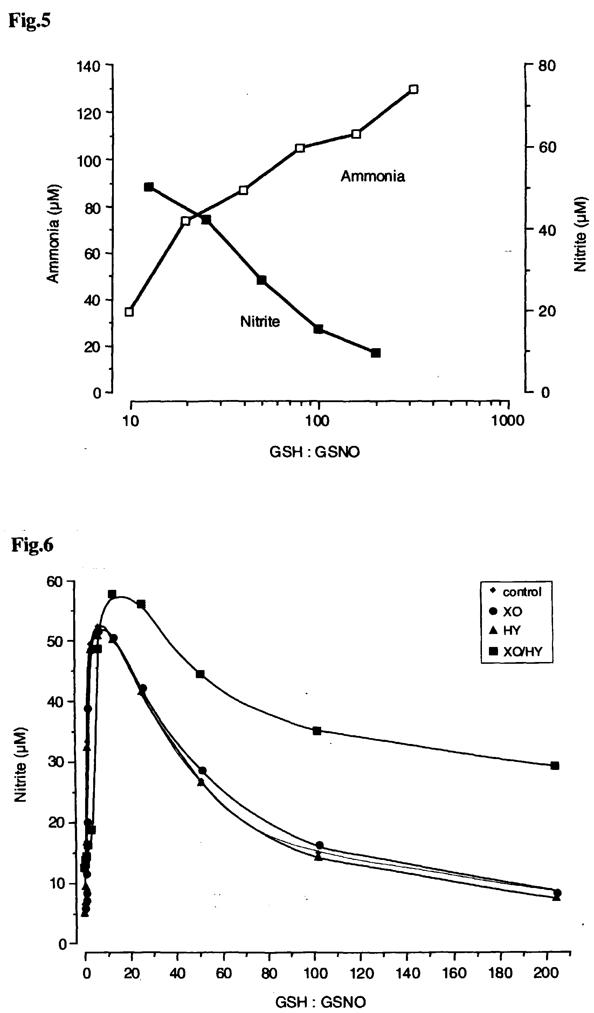

A role of ^{·}O₂⁻ in stimulating NO^{·} formation from S-nitrosothiols, emerged from a series of experiments in which it was shown that thiols such as GSH and cysteine enhanced the ratio of IFN-γ pc/IL-4 pc pointing to a potentiation of a T_{H}1 response pattern. This effect was found in cultures of both Lewis and BN rats (Table 1) and can at least partly be explained by the fact that GSH and or CSH can react with peroxynitrite. Moreover, exposure of rats to low dose mercuric chloride was found to stimulate ROI production by splenic macrophages which was associated with an increase in nitrite formation in spleen cell cultures of both strains of rats. This ROI-mediated increase in nitrite formation from S-nitrosothiols could be reproduced in a cell-free system in vitro. In one view of the Rijswijk cycle it can be perceived as a cyclic chain reaction driven by an interaction between NO^{·} and ^{·}O₂⁻ and fuelled with GSH which produces sequentially peroxynitrite (ONOO⁻), GSNO and glutathionyl radicals (GS^{·}).
This cyclic chain reaction is presented in the reaction pathways depicted in Figure 7. ^{·}O₂⁻ will accelerate GSH oxidation thereby shifting the equilibrium of the reaction between GSH-N-hydroxysulfenamide (GS-NOH-SG)and the two radicals GS^{·} and GS-N^{·}-OH to the right thus favouring nitrite and NO^{·} formation in conformity with the data of Figure 6, which shows that ^{·}O₂⁻ stimulates nitrite formation from GSNO at all concentrations of GSH in the reaction mixture.
The clinical relevance of our findings is illustrated by the reported association between high serum levels of nitrite/nitrate, low proliferative responses of autoreactive T cells and rarity of T_{H}1-type of autoimmune diseases in many tropical rural populations.
The present invention is of importance in the design of novel strategies in the treatment of AIDS and a variety of other infectious and autoimmune diseases.

As used herein the term "scavenger" means a compound capable of reducing at least in part the propensity of another compound to interact in a certain process. Thus a scavenger of for instance peroxynitrite may be found in compounds that associate with peroxynitrite thereby reducing at least in part the propensity of peroxynitrite to take part in the Rijswijk cycle. Alternatively a scavenger of peroxynitrite may be a compound capable of decomposing or converting peroxynitrite, whereby the products of the decomposition or the conversion have at least a reduced capability of participating in a significant manner in the Rijswijk cycle.

### Examples

### Materials and Methods

### Rats

Female BN and Lewis rats at 8 to 12 weeks of age were used in all experiments. Animals were obtained from Harlan CPB (Zeist, The Netherlands) and housed in the animal care facility of our institute and maintained on standard laboratory chow and water *ad libitum*.
For some experiments, rats were injected twice with 1 mg HgCl₂/kg body weight by the intraperitoneal route with an interval of 48 h. Four repeated i.p. injections with this dose of HgCl₂ at 48 h intervals induce a T_{H}2-biased autoimmune syndrome in BN but not Lewis rats that becomes clinically evident (massive proteinuria) approximately 13 days after the first injection with HgCl₂ (16).

### Reagents and media

Glutathione (G-4251), superoxide dismutase (S-5395), xanthine oxidase (X-1875), hypoxanthine (H-9377), phorbol 12-myristate 13-acetate (P-8139), ferricytochrome C (type III, C-2506), horseradish peroxydase (P-8250) and ammonia assay (171-B) were obtained from Sigma Chemical Co. (St. Louis, MO).
Concanavalin A (17-0450-01), N^{G}-monomethyl-L-arginine (475886), catalase (219008) and homovanillic acid (53620) were purchased from Calbiochem (La Jolla, CA).
Nitrate/nitrite assay was from Cayman Chem. Co. (Ann Arbor, MI).
Recombinant rat IFN-γ and mono- and polyclonal antibodies specific for rat IFN-γ were obtained from Genzyme Corp. (Cambridge, MA). The monoclonal antibody OX-81 (specific for rat IL-4) was a kind gift of Prof. D.W. Mason (MRC Cellular Unit, University of Oxford, UK). Polyclonal antibodies to rat IL-4 were generated in rabbits using E. coli produced recombinant rat IL-4 and purified by prot. A-affinity chromatography.
Both splenocytes and peritoneal macrophages (pM⌀) were cultured in RPMI-1640 medium (Gibco BRL) supplemented with 2 mM glutamine, 60 µg/ml streptomycin, 100 U/ml penicillin and 5% (CM5) or 10% (CM10) heat-inactivated foetal calf serum.
S-nitrosoglutathione (GSNO) was synthesised essentially as described by Hart (47). Purity was confirmed by HPLC analysis.

### Spleen cell preparation and culture

The aseptically removed spleen was gently crushed with the blunt end of a 5-ml syringe plunger and passaged through a fine mesh nylon screen. Erythrocytes were hemolysed by adding cold sterile water for 20 sec. to cell pellets followed by the addition of half the volume of 2.7% saline. The number of cells was determined by use of Türk's solution and cell viability by means of trypan blue exclusion. Cell suspensions in which the viability was less than 85% were excluded from the studies.
Splenocytes were washed twice with CM5, adjusted to 4 x 10⁶ cells/ml, transferred to a 24-well culture plate (1.0 ml/well) and stimulated with Con A (5 µg/ml) in the presence or absence of various supplements (as indicated in the text) for 40 h at 37°C in a humidified atmosphere containing 5% CO₂. Thereafter, non-adherent cells were collected and subjected to ELISPOT analyses as described below.

### Enumeration of cytokine secreting cells

The ELISPOT assay for detecting single cytokine secreting cells has been detailed previously (17). Briefly, the wells of PVC microplates were coated with monoclonal antibodies specific for rat IFN-γ or rat IL-4. After washing and postcoating, 100 µl suspensions of Con A-activated splenocytes were pipetted into the wells. The cells were allowed the produce cytokines for either 4 h (IFN-γ pc) or 16 h (IL-4 pc). Thereafter, cells were lysed for 10 minutes with ice-cold distilled water. Subsequently, the wells were thoroughly washed with PBS containing 0.05% Tween-20. Spots were detected by the sequential incubation of biotinylated rabbit anti-cytokine antibodies and goat anti-biotin antibodies conjugated to alkaline phosphatase (AP). The AP substrate 5-bromo-4-chloro-3-indolyl phosphate was used to produce blue zones ('spots') that visualised the positions of the individual spot-forming cells (SFC). SFC were enumerated by image analysis using a stereo microscope coupled to a colour video camera and Micro Image software from Olympus Optical Co. The frequency of spontaneously occurring SFC was always less than 1% of the mitogen-induced frequency.

### Measurement of H₂O₂ release by splenocytes

The production of H₂O₂ by splenocytes was measured spectrofluorometrically by the horse radish peroxidase (HRP)-mediated H₂O₂-dependent oxidation of homovanillic acid (18).
Splenocyte preparations were washed twice with PBS containing 550 mM D-glucose, 120 mM CaCl₂, 110 mM MgCl₂ and 5 mM Hepes buffer (PBS-G). Cells were resuspended at 10⁷ nucleated cells/ml in PBS-G to which 3 units HRP/ml and 100 nM homovanillic acid were added. Cell suspensions with or without stimuli were incubated at 37°C for 3-4 hours in 24-well plastic tissue culture plates (1.0 ml/well) in a humidified atmosphere containing 7% CO₂. The reaction was terminated with 0.25 ml 0.1 M glycine-NaOH buffer (pH12) containing 25 mM EDTA. The reaction mixture was transferred to plastic tubes, centrifuged at 10,000 x g to remove cell debris, and the amount of oxidised homovanillic acid was determined with a Perkin-Elmer Model 3000 spectrofluorometer.
Excitation was performed at 312 nm and emission recorded at 420 nm. All measurements were performed in triplicate and the results expressed as nmol H₂O₂ per 10⁷ splenocytes per hour.
Specific H₂O₂ release was determined after subtracting values from identical cell cultures which were kept at 0°C during the incubation of samples at 37°C. The spectrofluorometric values of wells containing all reagents plus a known amount of H₂O₂ were used to calculate the H₂O₂ released by splenocytes.

### Measurement of H₂O₂ production by peritoneal macrophages

The spontaneous or PMA/IFN-γ-triggered release of H₂O₂ was determined by the same method as described for splenocytes.
Macrophage monolayers were incubated for 24 h in CM10 with or without rat IFN-γ (1000 U/ml). Subsequently, cells were washed twice with prewarmed PBS and incubated for 1 h in 1.0 ml PBS containing 120 mM CaCl₂, 110 mM MgCl₂, 550 mM D-glucose, 1.0 unit HRP, 100 nM homovanillic acid and 5 ng/ml PMA. After termination of the reaction, the amount of oxidised homovanillic acid in the supernatant was determined spectrofluorometrically. The cellular proteins in the wells were solubilized by adding 250 µl 0.5% (w/v) sodium dodecylsulphate in 0.2 N NaOH. The amount of solubilized protein was determined by a modification of the Lowry method (19). The results are expressed as nmoles H₂O₂ per mg of protein.

### Isolation and culturing of peritoneal macrophages

Peritoneal cells were collected from rats by lavage with 10 ml ice-cold saline containing 50 units/ml heparin. Cells were washed twice with CM10 and resuspended in CM10 to a concentration of 2 x 10⁶ cells/ml. Total cell counts were obtained with a Bürker hemocytometer and differential counts performed on centrifuge preparations fixed in absolute methanol and stained with Giemsa stain. The peritoneal cell suspension of Lewis rats consisted of 70 ± 3% macrophages, 13 ± 1% lymphocytes, 14 ± 1% granulocytes and 3 ± 1% mast cells (n = 3). The peritoneal cells of BN rats were composed of 61 ± 2% macrophages, 12 ± 1% lymphocytes, 26 ± 2% granulocytes and 2 ± 1% mast cells (n = 3).
Cells were fractionated by culturing them in the wells of a 24-well tissue culture plate at a concentration of 2 x 10⁶ cells/ml in CM10. After 2 h incubation at 37°C in a humidified atmosphere containing 7% CO₂, the nonadherent cells were removed by three washes and the adherent cells, consisting of more than 95% of macrophages were used for studying the spontaneous and PMA/IFN-γ stimulated release of ROI.

### Measurement of superoxide anions

The pMØ monolayers were incubated for 20 h at 37°C in CM10 and subsequently washed (twice) with Hanks' balanced salt solution (HBSS) without phenol red. Thereafter, the cells were treated with PMA and assayed for ^{·}O₂⁻ production by measuring the rate of SOD-inhibitable ferricytochrome C reduction (20, 21). To that purpose the macrophage monolayer was incubated for 1 h at 37°C with an 1.0 ml reaction mixture containing 100 µM ferricytochrome C in HBSS with or without PMA (100 ng/ml). Reference samples additionally contained 50 µg/ml SOD. Each test was run in duplicate and the absorption increase at 550 nm was measured using a Beckmann DU-5 spectrophotometer. ^{·}O₂⁻ release by splenocytes was determined after Con A activation of 4 x 10⁶ cells for 20 h. The cells were washed twice with HBSS without phenol red and assayed for ^{·}O₂⁻-release essentially as described for the pMØs.

### Generation of ROI by xanthine oxidase and hypoxanthine

Xanthine oxidase (XO) can react with the purine substrate hypoxanthine (HY) to produce superoxide anions and hydrogen peroxide *in vitro* (22). Before this ROI generating system was used in our experiments, the optimal concentration of both XO and HY was determined by checkerboard titrations. These experiments revealed that XO up to 5 mU/ml and 0.1 mM HY added to cultures of BN spleen cells in concert with Con A did not affect cell viability as assessed by trypan blue exclusion. However, the addition of 10 mU/ml XO induced a significant decrease in the number of viable cells. Based on these findings we used 5 mU/ml XO and 0.1 mM HY throughout this study in both the cell-free systems and splenocyte cultures.

### Statistics

If appropriate, statistical significance of differences between the results obtained with cells of the two rat strains was determined by the Mann-Whitney U test.
Differences of P < 0.05 were considered significant.

### Example 1

### T_{H}1/T_{H}2 cytokine response patterns

We first determined the frequency of T cells secreting IFN-γ and IL-4 in cultures of mitogen-stimulated rat spleen cells.
In this process, macrophages are known to play a critical modulatory role (23, 24).
We chose for Con A as stimulus since the profile of cytokine production by naive and memory T cells in response to this mitogen has shown to be very similar to that observed with alloantigen stimulation (25). Because the differentiation of CD4⁺ T cells towards a T_{H}1 or T_{H}2 phenotype *in vitro* and *in vivo* takes place in less than 48 h (26), we decided to stimulate the cells for 40 h with Con A and then to enumerate IFN-γ pc and IL-4 pc in the non-adherent cell fraction by ELISPOT analyses. As shown in Table 1, BN splenocytes generate far more IL-4 pc than those of Lewis rats, whereas IFN-γ pc occur at a higher frequency in cultures of Lewis splenocytes. This differential effect is particularly evident when we express the data in the ratio of IFN-γ pc/IL-4 pc (Table 1).
The addition of excess glutathione (GSH) or other reducing agents such as cysteine to the cultures enhanced the ratio of IFN-γ pc/IL-4 pc approximately 10-fold irrespective whether the splenocytes originated from BN or Lewis rats (Table 1).
This stimulation of a T_{H}1 response pattern was attributed to both an increase in IFN-γ pc and a decrease in IL-4 pc. In spite of this strong stimulatory effect of GSH on T_{H}1 type of responses, the ratio of IFN-γ pc/IL-4 pc was consistently lower (approximately 7-fold) in BN as compared to Lewis spleen cell cultures. It should be noted that the data also revealed a strong inverse relationship in spot-size between IFN-γ pc and IL-4 pc indicating that cytokine production at the single cell level was reciprocally affected by GSH (see below).

### Example 2

### In vivo exposure to HgCl₂

BN rats are highly susceptible for a T_{H}2-driven autoimmune syndrome induced by certain drugs and chemicals such as penicillamine and HgCl₂ (11, 27). Lewis rats, on the other hand, are not susceptible for this disorder, but show high susceptibility for T_{H}1-associated autoimmune diseases such as experimental autoimmune encephalomyelitis (EAE) and adjuvant arthritis (AA) to which BN rats are resistant (28-30). To study the effect of HgCl₂ on the generation of IFN-γ pc and IL-4 pc, we analysed splenocytes from both Lewis and BN rats at day 3 after the first injection with HgCl₂ (2 injections with a 48 h interval) which is approximately nine days before clinical signs of disease become evident in BN rats. These analyses revealed an approximate 6-fold reduction in the ratio of IFN-γ pc/IL-4 pc in BN and a 2-fold reduction in Lewis splenocyte cultures (Table 2).
In the presence of excess GSH (2 mM), the ratio of IFN-γ pc/IL-4 pc in spenocyte cultures of HgCl₂-exposed BN rats was approximately 25-fold lower than the ratio in cultures of HgCl₂-exposed Lewis rats in conformity with a strong polarisation towards a T_{H}2-type of response in BN rats (Table 2).

### Example 3

### Redox status macrophages

A critical modulatory role for macrophages in polarising T_{H} subset differentiation can be envisaged at several levels.
Macrophages may differentially express co-stimulatory molecules involved in promoting IL-4 production (e.g. CD40; 31), show differences in the production of T cell modulatory cytokines (e.g., IL-12 and IFN-α; 32, 33) and/or vary in their redox status affecting particular T cell functions (34, 35). In this study we focused on the latter possibility by measuring the production of H₂O₂ and superoxide anions (^{·}O₂⁻) by polyclonally stimulated spleen cells and by highly purified peritoneal macrophages (pMØs) of both BN and Lewis rats. These experiments showed that the spontaneous and ConA-induced release of H₂O₂ was approximately 3-fold higher in BN than in Lewis spleen cell cultures. The ^{·}O₂⁻ production, on the other hand, was too low to be measured. With respect to pMØs, cells from BN rats produced 3- to 7-fold higher amounts of both H₂O₂ and ^{·}O₂⁻ than those from Lewis rats (Table 3).
Since adherent splenic cells showed a similar strain-specific difference in H₂O₂ production, macrophages are most likely responsible for the enhanced production of H₂O₂ in the BN spleen.
*In vivo* administration of HgCl₂ caused a 2- to 3-fold enhancement of H₂O₂ production by splenocytes from both strains at 72 h after the first injection with HgCl₂ (not shown). ^{·}O₂⁻ levels, however, were again below the detection limit of our assay.

### Example 4

### Role of ROI in T_{H} subset differentiation

To examine a putative role for ROI in the generation of IFN-γ pc and IL-4 pc, we either enhanced or scavenged H₂O₂ and ^{·}O₂⁻during Con A stimulation of splenocytes derived from normal and HgCl₂-exposed rats. Since ROI have been reported to play a stimulatory role on T cell activation at a very early time point (34), we determined the effects of ROI scavengers (catalase and SOD) and ROI species (H₂O₂ and ^{·}O₂⁻) when added at different time points after initiation of Con A stimulation (0, 6, 20 and 30 h) . It was found that the T_{H}1/T_{H}2 response pattern was unaffected by catalase. Neither the time of addition nor whether catalase (up to 900 U/ml) was added to cultures from normal or HgCl₂-exposed rats revealed any modulatory effect. Also the addition of high levels of H₂O₂ (up to 100 µM) to the cultures did not affect the frequencies of IFN-γ pc and IL-4 pc (not shown).
SOD, on the other hand, produced a dramatic effect lowering the ratio of IFN-γ pc/IL-4 pc 10- to 30-fold in cultures of both naive and HgCl₂-exposed BN rats (Table 4). Although SOD showed strong modulatory effects when added at various time points after initiation of mitogen stimulation, optimal results were obtained when SOD was added at 20 h. Similar effects were found in Lewis spleen cell cultures, although 2-fold higher concentrations of SOD were needed to reach a comparable reduction (more than 10-fold) in the ratio of IFN-γ pc/IL-4 pc (Table 4).
Interestingly, SOD significantly augmented the generation of IL-4 pc particularly in Lewis spleen cell cultures (Table 4).
We subsequently enhanced ^{·}O₂⁻ production by adding an ^{·}O₂⁻generating system (xanthine oxidase plus hypoxanthine [XO/HY], 36) to splenocyte preparations from naive Lewis rats. The XO/HY system was added 20 h after initiation of Con A stimulation. As shown in Table 5, this exogenous source of ^{·}O₂⁻ strongly inhibited the generation of IFN-γ pc and slightly enhanced the frequency of IL-4 pc. XO/HY thus redirected the T_{H}1 response towards a profound T_{H}2 one in Lewis spleen cell cultures.
At first glance, these observations appear contradictory. Whereas scavenging of ^{·}O₂⁻ promotes T_{H}2 responses, enhancement of ^{·}O₂⁻ levels produced a similar effect suggesting the involvement of two opposing mechanisms of action.

### Example 5

### Nitric oxide

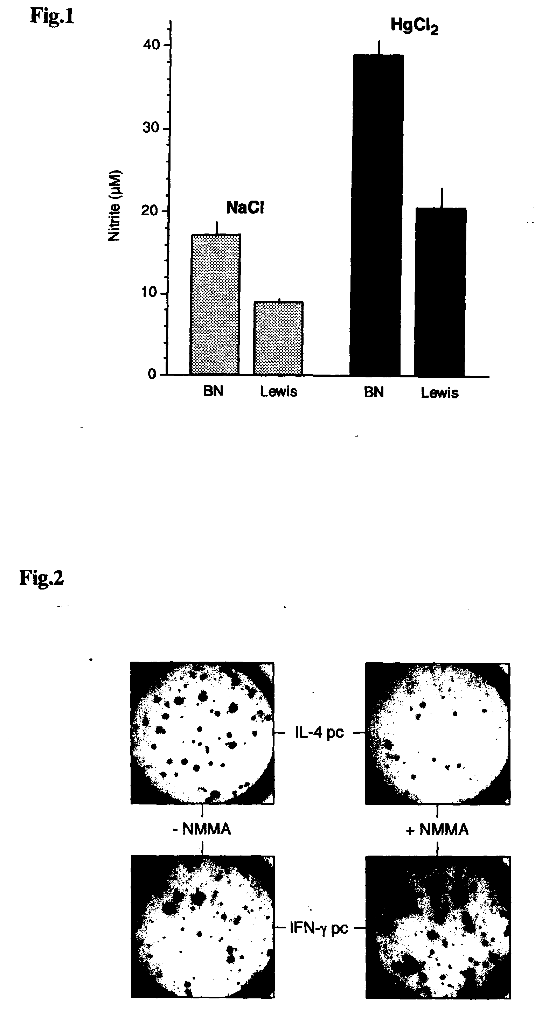

One possible explanation for the inhibitory effect of SOD on T_{H}1 cytokine response pattern is an increase in NO^{·} bioactivity which may selectively inhibit the generation of IFN-γ pc as reported by us previously (16).NO^{·} is abundantly produced by macrophages at 12 to 48 h after stimulation (37) and is known to rapidly interact with ^{·}O₂⁻ to produce peroxynitrite (rate constant ∼ 6.7 x 10⁹ M⁻¹S⁻¹; 38) thereby neutralising the activity of both radicals (39, 40). A critical role for NO^{·} is also in accordance with our findings that endogenous erythrocytes enhance the ratio of IFN-γ pc/IL-4 pc 2- to 3-fold in splenocyte cultures of both strains. This effect can be explained either by the rapid interaction of NO^{·} with the iron-containing heme group of deoxyhemoglobin or by the oxidative reaction between NO^{·} and O₂ of oxyhemoglobin to form nitrate (41). Moreover, the addition of an excess of purified red blood cells (RBCs) to Con A-stimulated splenocytes from normal BN rats enhanced the generation of IFN-γ pc approximately 5-fold and simultaneously reduced the accumulation of nitrite, a measure for NO^{·} production, more than 3-fold (not shown).
A role for NO^{·} is also suggested by our findings that splenocyte cultures of BN rats contained approximately 2-fold more nitrite than those of Lewis rats at 48 h post-initiation Con A stimulation which is increased 2- to 3-fold in cultures from HgCl₂-exposed rats (Fig. 1).

When we added a competitive inhibitor of NO^{·} synthesis (N^{G}-monomethyl-L-arginine [NMMA]) to splenocyte cultures of naive BN rats, there was a 4-fold increase in the ratio of IFN-γ pc/IL-4 pc. In cultures of HgCl₂-exposed rats, NMMA enhanced the ratio of IFN-γ pc/IL-4 pc 11-fold in BN and 2-fold in Lewis splenocyte cultures (Table 6). As illustrated in Figure 2, NMMA also had a major impact on spot-size, which point to an effect of NO^{·} on cytokine production by individual cells as well.

### Example 6

### S-nitrosothiols

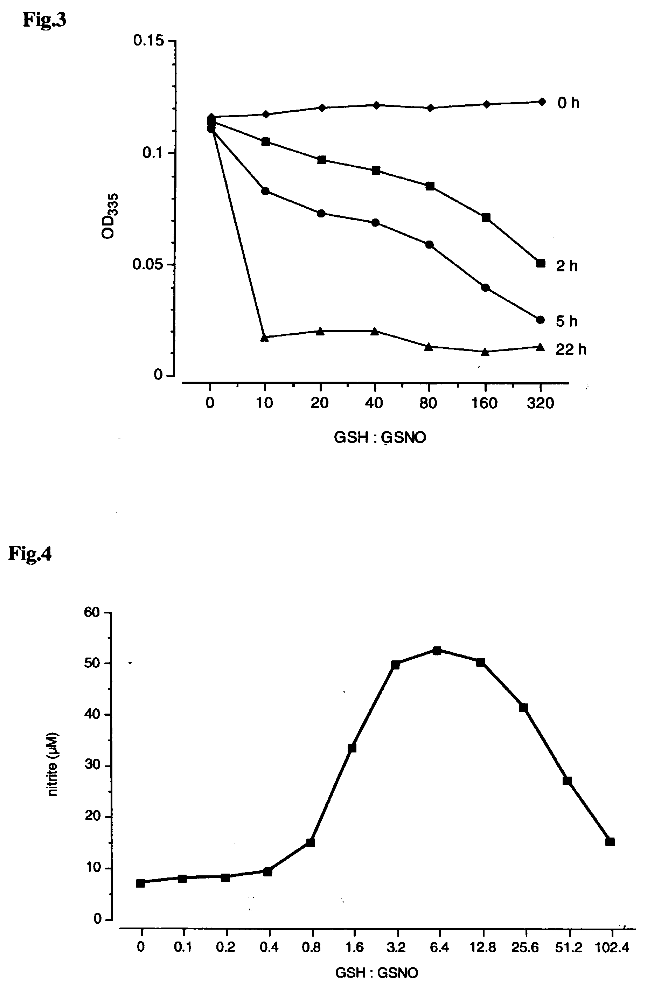

It has been shown that the unequal formation of ^{·}O₂⁻ and NO^{·} during cell culturing is associated with apoptotic and/or necrotic cell death (42), suggesting that the simultaneous production of ^{·}O₂⁻ and NO^{·} in a balanced ratio reflects a protective principle antagonising the destructive actions of the two radicals when acting individually.
Since it has been reported that T_{H}1 cells are far more susceptible to apoptosis than T_{H}2 cells (43), ^{·}O₂⁻ production in excess of NO^{·} may explain the preferred T_{H}2 response in BN splenocyte cultures. In such a concept, inhibition of NO^{·} synthesis would increase the imbalance in the levels of ^{·}O₂⁻and NO^{·} thereby potentiating T_{H}2 responses. However, NMMA showed the opposite effect and potentiated T_{H}1 responses (Table 6) arguing against this possibility.
To balance NO^{·} and ^{·}O₂⁻ production, it can be assumed that regulatory mechanism(s) have evolved in vivo to control the production of both radicals. Since there is evidence that the production of ROT and RNI is independently regulated (44) and the fact that we were unable to detect significant differences in the production of both iNOS mRNA and nitrite by pMØs of BN and Lewis rats, secondary mechanism(s) may be involved. One attractive possibility is the involvement of NO donors such as S-nitrosocysteine (CSNO) and S-nitroso-glutathione (GSNO) which can rapidly supply NO^{·} to match the production of ^{·}O₂⁻ (45, 46). We reasoned that ^{·}O₂⁻ may affect GSNO/CSNO decomposition resulting in an enhancement of NO^{·} formation and as such provide an explanation for the higher levels of nitrite in BN vs. Lewis spleen cell cultures.
In a series of experiments using synthetically produced GSNO dissolved in a phosphate buffer as a model system, we examined the effect of increasing amounts of GSH and ROI on the decomposition of GSNO as measured by the decline in absorbance at 335 nm (47). Additionally, we determined the amounts of nitrite, nitrate and ammonia that was formed during decomposition. Nitrite and ammonia have recently been shown to be the two major nitrogen-containing metabolites formed during GSH-triggered decomposition of GSNO (45). In the absence of GSH, GSNO was found to be very stable and no significant decomposition took place when 0.1 mM of the compound was incubated for 42 h at room temperature (not shown). The subsequent addition of GSH induced a rapid breakdown of GSNO, the rate of which was positively correlated with the concentration of GSH. At a ratio of GSH:GSNO of 320, decomposition was almost completed in 5 h (fig. 3). Nitrite formation was maximum at molar ratios ranging from 3 to 10 and declined rapidly when GSH was present in large molar excess (fig. 4). At molar ratios exceeding 20, the major decomposition product was NH₃ (Fig. 5). Nitrate was not detected in the solutions. To examine the effect of ROI on the breakdown of GSNO, we added either H₂O₂ or XO/HY to the GSNO solutions containing various concentrations of GSH. No significant modulatory effect of H₂O₂ on both GSNO decomposition and nitrite formation was found. XO/HY, on the other hand, produced a major increase in the accumulation of nitrite (Fig. 6) and slightly accelerated the rate of GSNO decomposition indicating that ^{·}O₂⁻ promotes a pathway of GSNO decomposition that preferentially generates nitrite.

### Example 7

### Dose finding study in rhesus monkeys

To find suitable dose for the administration of peroxynitrite scavengers a test range of different amounts of the compound was given to different rhesus monkeys. Monkeys that received PBS served as control monkeys. Monkeys, both male and female, were selected that were approximately 2 to 3 years old.
The dose range for FeTMPS was from 10 mg/kg body weight (bw) till 100 mg/kg b.w.
The compound, solubilised in 3.0 ml phosphate buffered saline, was injected in the upper part of both hind legs.
The doses were given subcutaneously once daily for 10 days in a row.
The dose range for uric acid was from 1,0 g/kg b.w. till 50 gram/kg b.w. The doses were given subcutaneously once daily for 10 days in a row.
The monkeys were monitored by clinical observation, clinical chemistry and hematology.
Blood samples were taken 5 minutes before each administration and at different time points (5 min., 15 min., 30 min., 60 min., 2 hours, 4 hours, 8 hours, and 23 hours after each administration).

Samples were analysed for the test substance to determine pharmacokinetics and for the ammonia/nitrite content and ratio.

### Example 8

### Efficacy of peroxynitrite scavenger.

Suitable dose ranges were selected on the basis of the acceptable clinical side effects, acceptable clinical chemistry values and acceptable hematology side effects.
Twelve healthy rhesus monkeys at 2 to 4 years of age (6 males and 6 females) were infected with simian immunodeficiency virus (SIV) strain 251. Six monkeys were treated with test substance whereas other monkeys received control substance.
Based on the data of the dose finding study/toxicity study, a suitable peroxynitrite scavenger was selected for the efficacy study. The optimal dose of this selected compound was given to the monkeys in 3.0 ml phosphate buffered saline per injection site. The injections were given alternatively in the left and in the right upper part of both hind legs.
Treatment with the test and control substance was started on the day of infection with the SIV virus. Treatment continued until 3 control monkeys developed AIDS-like symptoms. Monkeys were considered to have AIDS-like symptoms when they exhibited one or more of the following symptoms. Cachexia, diarrhea or pneumonia in conjunction with a decrease, compared to the number before the start of the experiment, in the number of CD4⁺ lymphocytes in the peripheral blood for a prolonged period of time.
Biweekly blood samples from monkeys were further analysed for hematology, clinical chemistry, neopterin, nitrate, nitrite, ammonia, CD4⁺ lymphocytes, pathology, necropsis.

### Tables and figures

**Table 1**

| **The effect of GSH on the Con A-induced generation of IF**N-γ **pc and IL-4 pc in spleen cell cultures of normal rats** | | | | |
|---|---|---|---|---|
| rat Strain | culture const. | IL-4 pc (per 2 x 10⁵ MNC) | IFN-γ pc (per 1 x 10⁴ MNC) | ratio IFN-γ pc/IL-4 pc |
| BN | GSH(2 mM) | 60 ± 5 | 98 ± 11 | 34 |
| BN | no GSH | 122 ± 6 | 15 ± 2 | 2.5 |
| Lewis | GSH(2 mM) | 11 ± 3 | 139 ± 14 | 253 |
| Lewis | no GSH | 37 ± 10 | 21 ± 3 | 11 |

The generation of both IFN-γ PC and IL-4 pc in the absence of ConA was less than 1% of the presented frequencies. The experiments were performed at four independent occasions with similar results. At each occasion the ELISPOT assay was performed in quadruplicate. One representative experiment is presented. Both enhancement of IFN-γ pc and reduction of IL-4 pc by GSH were in all analyses statistically significant (p < 0.01).

**Table 2**

| ***In vivo*** **exposure to HgCl**_{**2**} **promotes T**_{**H**}**2-type of responses in rat spleen cell cultures** | | | | |
|---|---|---|---|---|
| rat strain | *in vivo* administration | IL-4 pc (per 2 x 10⁵ MNC) | IFN-γ pc (per 1 x 10⁴ MNC) | ratio IFN-γ pc/IL-4 pc |
| BN | saline | 56 ± 9 | 104 ± 21 | 37 |
| BN | HgCl₂ | 101 ± 12 | 34 ± 8 | 6.7 |
| Lewis | saline | 9 ± 5 | 144 ± 16 | 320 |
| Lewis | HgCl₂ | 12 ± 7 | 103 ± 9 | 172 |

Spleens were isolated from rats at 24 h after the second intraperitoneal injection with saline or HgCl₂ (1 mg/kg b.w.). Spleen cells were stimulated with Con A (5 µg/ml) in the presence of 2 mM GSH. The experiments were performed at three independent occasions with similar results. At each occasion the ELISPOT analyses were performed in quadruplicate. One representative experiment is presented. Both reduction of IFN-γ pc and enhancement of IL-4 pc (BN splenocytes only) as a result of *in vivo* exposure to HgCl₂ reached in all analyses statistical significance (p < 0.01).

**Table 3**

| **ROI production by purified peritoneal macrophages from normal rats** | | | |
|---|---|---|---|
| strain | *in vitro* stimulus | ROI | |
| | | H₂O₂ (nmol/mg protein) | ^{·}O₂⁻ (nmol/mg protein) |
| BN | none | 0.2 | 13.9 |
| BN | IFN-γ (10³ U/ml) | 0.9 | 2.7 |
| BN | PMA (5 ng/ml) | 8.7 | 388.2 |
| Lewis | none | 0.0 | 1.8 |
| Lewis | IFN-γ (10³ U/ml) | 0.0 | 0.0 |
| Lewis | PMA (5 ng/ml) | 1.9 | 62.1 |

Peritoneal macrophages (pMØs) were isolated and stimulated as described in the Materials and Methods section.
Five independent experiments were performed providing essentially similar results. The outcome of one representative experiment is shown. Differences in H₂O₂ and ^{·}O₂⁻ production by macrophages from BN and Lewis rats were statistical significant (p < 0.01).

**Table 4**

| **SOD promotes T**_{**H**}**2-type of responses in rat spleen cell cultures** | | | | | |
|---|---|---|---|---|---|
| rat strain | *in vivo* administration | culture supplements | IL-4 pc per 2 x 10⁵ MNC | IFN-γ pc per 1 x 10⁴ MNC | ratio IFN-γ pc/IL-4 pc |
| BN | saline | none | 51 ± 14 | 90 ± 7 | 35 |
| BN | saline | SOD (80 U/ml) | 67 ± 5 | 51 ± 4 | 15 |
| BN | saline | SOD (160 U/ml) | 72 ± 9 | 11 ± 5 | 3 |
| Lewis | saline | none | 8 ± 2 | 126 ± 14 | 315 |
| Lewis | saline | SOD (160 U/ml) | 14 ± 5 | 71 ± 11 | 101 |
| Lewis | saline | SOD (320 U/ml) | 20 ± 3 | 12 ± 8 | 12 |
| BN | HgCl₂ | none | 88 ± 14 | 30 ± 8 | 6.8 |
| BN | HgCl₂ | SOD (80 U/ml) | 82 ± 7 | 11 ± 3 | 2.7 |
| BN | HgCl₂ | SOD (160 U/ml) | 83 ± 10 | 1 ± 1 | 0.2 |
| Lewis | HgCl₂ | none | 11 ± 3 | 104 ± 1 | 189 |
| Lewis | HgCl₂ | SOD (160 U/ml) | 19 ± 4 | 63 ± 10 | 66 |
| Lewis | HgCl₂ | SOD (320 U/ml) | 26 ± 4 | 8 ± 5 | 6.2 |

Splenocytes from both normal and HgCl₂-exposed rats were examined. Cells were stimulated for 20 h with Con A (5 µg/ml) in cultures containing 2 mM GSH. After this initial stimulation step, SOD was added at the indicated concentrations and incubation was continued for another 20 h.
Thereafter the non-adherent cells were collected and subjected to ELISPOT analyses. Four independent experiments using in total 8 saline- and 8 HgCl₂-treated rats were performed. The results of one representative experiment in quadruplicate is presented. The SOD-mediated decrease in IFN-γ pc in both BN and Lewis spleen cell cultures reached in each independent experiment statistical significance (p < 0.01).

**Table 5**

| **An exogenous source of superoxide anions promotes T**_{**H**}**2-type of responses in Lewis spleen cell cultures** | | | |
|---|---|---|---|
| culture constituents | IL-4 pc (per 2 x 10⁵ MNC) | IFN-γ pc (per 1 x 10⁴ MNC) | ratio IFN-γ pc/IL-4 pc |
| GSH (2 mM) | 6 ± 1 | 103 ± 23 | 343 |
| GSH (2 mM) + XO/HY | 12 ± 2 | 61 ± 11 | 102 |
| none | 35 ± 6 | 23 ± 6 | 13 |
| XO/HY | 40 ± 7 | 1 ± 1 | <1 |

Splenocytes from normal Lewis rats were examined. Cells were stimulated with Con A (5 µg/ml) in the presence or absence of GSH. After 20 h of incubation, 5 mU/ml xanthine oxidase plus 0.1 mM hypoxanthine (XO/HY) was added. Incubation was continued for another 20 h before the non-adherent cells were collected and subjected to ELISPOT analyses. Two independent experiments were executed with similar results. ELISPOT analyses were performed in fivefold. The results of one experiment are presented. The XO/HY-mediated decrease in IFN-γ pc reached in both experiments statistical significance (p < 0.01).

**Table 6**

| **Inhibition of NO synthesis favours T**_{**H**}**1-type of responses in rat spleen cell cultures** | | | | | |
|---|---|---|---|---|---|
| rat strain | *in vivo* administration | culture supplements | IL-4 pc per 2x10⁵ MNC | IFN-γ pc per 1x10⁴ MNC | ratio pc IFN-γ/IL-4 |
| BN | none | none | 50 ± 4 | 108 ± 7 | 43 |
| BN | none | NMMA (180 µM) | 32 ± 6* | 209 ± 19* | 131 |
| BN | none | NMMA (360 µM) | 30 ± 2* | 251 ± 14* | 167 |
| Lewis | none | none | 12 ± 4 | 182 ± 16 | 303 |
| Lewis | none | NMMA (180 µM) | 14 ± 7 | 175 ± 9 | 250 |
| Lewis | none | NMMA (360 µM) | 13 ± 2 | 178 ± 11 | 274 |
| BN | HgCl₂ | none | 87 ± 14 | 35 ± 8 | 8 |
| BN | HgCl₂ | NMMA (180 µM) | 54 ± 11* | 71 ± 11* | 26 |
| BN | HgCl₂ | NMMA (360 µM) | 20 ± 5* | 92 ± 17* | 92 |
| Lewis | HgCl₂ | none | 13 ± 3 | 104 ± 11 | 160 |
| Lewis | HgCl₂ | NMMA (180 µM) | 10 ± 5 | 112 ± 5 | 224 |
| Lewis | HgCl₂ | NMMA (360 µM) | 8 ± 2* | 135 ± 10* | 338 |

| | | | | | |
|---|---|---|---|---|---|
| *indicates significant differences in cytokine producing cells (p < 0.05) between cultures with or without NMMA. | | | | | |

Splenocytes from both naive and HgCl₂-exposed rats were examined. Cells were stimulated with Con A (5 µg/ml) in the presence of 2 mM GSH.

After 20 h, N^{G}-monomethyl-L-arginine (NMMA) was added at the indicated concentrations and incubation was continued for another 20 h.
Thereafter the non-adherent cells were collected and subjected to ELISPOT analyses. Three independent experiments were performed. During each experiment splenocytes from one naive and one HgCl₂-exposed rat of both strains were examined. The results of one representative experiment in quadruplicate is presented.

### Abbreviations

ROI, reactive oxygen intermediates; RSNO, S-nitrosothiols; GSNO, S-nitrosoglutathione; CSNO, S-nitrosocysteine; XO/HY, xanthine oxidase plus hypoxanthine; GSH, glutathione; CSH, cysteine; pc, producing cells; BN, Brown Norway; SOD, superoxide dismutase; ELISPOT, enzyme-linked immunospot; RNI, reactive nitrogen intermediates; NNMA, N^{G}-mono-methyl-L-arginine; iNOS, inducible nitric oxide synthase; Con A, concanavalin A; MNC, mononuclear cells.

### References

1. Constant, S.L. and K. Bottomly. 1997. Induction of T_{H}1 and T_{H}2 CD4⁺ T cell responses: The alternative approaches. Annu. Rev. Immunol. **15**: 297-322.
2. Nakamura, T., R.K. Lee, S.Y. Nam, E.R. Podack, K. Bottomly and R.A. Flavell, 1997. Roles of IL-4 and IFN-γ in stabilizing the T helper cell type 1 and 2 phenotype. J. Immunol. **158**: 2648-2653.
3. Gorham, J.D., M.L. Guler, R.G. Steen, A.A. MacKey, M.J. Daly, K. Frederick, W.F. Dietrich and K.M. Murphy. 1996. Genetic mapping of a murine locus controlling development of T helper 1/T helper 2 type responses. Proc. Natl. Acad. Sci. U.S.A. **93**: 12467-12472.
4. Hosken., N.A., K. Shibuya, A.W. Heath, K.M. Murphy and A. O'Garra. 1995. The effect of antigen dose on CD4⁺ T helper cell phenotype development in a T cell receptor-ab transgenic model. J. Exp. Med. **182**: 1579-1584.
5. Coffman, R.L. and T.Von der Weid. 1997. Multiple pathways for the initiation of T helper 2 (T_{H}2) responses. J. Exp. Med. **185**: 373-375.
6. Secrist, H., R.H. DeKruijff and D.T. Umetsu. 1995. Interleukin 4 production by CD4⁺ T cells from allergic individuals is modulated by antigen concentration and antigen-presenting cell type. J. Exp. Med. **181**: 1081-1089.
7. Macatonia, S., N.A. Hosken, M. Litton, P. Vieira, C.-S. Hsieh, J.A. Culpepper, M. Wysocka, G. Trinchieri, K.M. Murphy and A. O'Garra.. 1995. Dendritic cells produce IL-12 and direct the development of T_{H}1 cells from naive CD4⁺ T cells. J. Immunol. **154**: 5071-5079.
8. Pfeiffer, C., J. Stein, S. Southwood, H. Ketelaar, A. Settle and K. Bottomly. 1995. Altered peptide ligands can control CD4 T lymphocyte differentiation *in vivo*. J. Exp. Med. **181**: 1569-1574.
9. Thompson, C.B. 1995. Distinct roles for the costimulatory ligands B7-1 and B7-2 in T helper cell diffferentiation. Cell **81**: 979-982.
10. Caspi, R.R. , P.B. Silver, C.C. Chan, B. Sun, R.K. Agarwal, J. Wells, S. Oddo, Y. Fujiro, F. Najafian and R.L. Wilder. 1996. Genetic susceptibility to experimental autoimmune uveoretinitis (EAU) in the rat is associated with an elevated T_{H}1 response. J. Immunol. **157**: 2668-2675.
11. Goldman, M., P. Druet and E. Gleichmann, 1991. T_{H}2 cells in systemic autoimmunity: insights from allogeneic diseases and chemically-induced autoimmunity. Immunol. Today **12**: 223-227.
12. Conboy, I.M., R.H. DeKruijff, K.M. Tate, Z.A. Cao, T.A. Moore, D.T. Umetsu and P.P. Jones. 1997. Novel genetic regulation of T helper 1 (T_{H}1)/T_{H}2 cytokine production and encephalitogenicity in inbred mouse strains. J. Exp. Med. **185**: 439-451.
13. Guéry, J.C. , F. Galbiati, S. Smiroldo and L. Adorini. 1997. Non-MHC-linked T_{H}2 cell development induced by soluble protein administration predicts susceptibility to Leishmania major infection. J. Immunol. **159**: 2147-2153.
14. Scott, B., R. Liblau, S. Degermann, L.A. Marconi, L. Ogata, A.J. Caton, H.O. McDevitt and D. Lo. 1994. A role for non-MHC genetic polymorphism in susceptibility to spontaneous autoimmunity. Immunity **1**: 73-82.
15. Lanzavecchia, A. 1997. Understanding the mechanisms of sustained signaling and T cell activation. J. Exp. Med. **185**: 1717-1719.
16. Van der Meide, P.H., M.C.D.C. de Labie, C.A.D. Botman, J. Aten and J.J. Weening. 1995. Nitric oxide suppresses IFN-γ production in the spleen of mercuric chloride-exposed Brown Norway rats. Cell. Immunol. **161**: 195-206.
17. Ruuls, S.R., M.C.D.C. de Labie, K.S. Weber, C.A.D. Botman, R.J. Groenestein, C.D. Dijkstra, T. Olsson and P.H. van der Meide. 1996. The length of treatment determines whether IFN-b prevents or aggravates experimental autoimmune encephalomyelitis in Lewis rats. J. Immunol. **157**: 5721-5731.
18. Ruch, W., P.H. Cooper and M. Baggiolini. 1983. Assay of H₂O₂ production by macrophages and neutrophils with homovanillic acid and horseradish peroxidase. J. Immunol. Meth. **63**: 347-357.
19. Markwell, M.A.K., S.M. Haas, L.L. Bieber and N.E. Tolbert. 1978. A modification of the Lowry procedure to simplify protein determination in membrane and lipoprotein samples. Anal. Biochem. **87**: 206-210.
20. Johnston Jr., B.B., B.B. Keele Jr., H.P. Misra, J.E. Lehmeyer, L.S. Webb, R.L. Baehner, and K.V. Rajagopalan. 1975. The role of superoxide anion generation in phagocytic bactericidal activity. J. Clin. Invest. **55**: 1357-1372.
21. Babior, B.M., R.S. Kipnes and J.T. Curnutte. 1973. Biological defense mechanisms: the production by leukocytes of superoxide, a potential bactericidal agent. J. Clin. Invest. **52**: 741-744.
22. Kimura, H. and M. Nakano. 1988. Highly sensitive and reliable chemiluminescence method for the assay of superoxide dismutase in human erythrocytes. FEBS Lett. **293**: 347-350.
23. Unanue, E.R. and P.M. Allen. 1987. The basis for the immunoregulatory role of macrophages and other cells. Science **236**: 551-557.
24. Desmedt, M., P. Rottiers, H. Dooms, W. Fiers and J. Grooten. 1998. Macrophages induces cellular immunity by activating T_{H}1 cell responses and suppressing T_{H}2 cell responses. J. Immunol. **160**: 5300-5308.
25. Akbar, A.N., M. Salmon and G. Janossy. 1991. The synergy between naive and memory T cells during activation. Immunol. Today **12**: 184-188.
26. Launois, P., K.G. Swihart, G. Milon and J.A. Louis. 1997. Early production of IL-4 in susceptible mice infected with Leishmania major rapidly induces IL-12 unresponsiveness. J. Immunol. **158**: 3317 -3324.
27. Tournade, H., L. Pelletier, R. Pasquier, M.C. Vial, C. Mandet and P. Druet. 1990. D-penicillamine-induced autoimmunity in Brown Norway rats. Similarities with HgCl₂-induced autoimmunity. J. Immunol. **144**: 2985-2991.
28. Gasser, D.L., C.M. Newlin, J. Palm and N.K. Gonatas. 1973. Genetic control of susceptibility to experimental allergic encephalomyelitis in rats. Science **181**: 872-873.
29. Matsumoto, Y., K. Kawai, Y. Tomita and M. Fujiwara. 1990. Limiting-dilution analysis of the frequency of myelin basic protein-reactive T cells in Lewis, PVG/c and BN rats. Implication for susceptibility to autoimmune encephalomyelitis. Immunology **69**: 215-221.
30. Ulmansky, R. and Y. Naparstek. 1995. Immunoglobulins from rats that are resistant to adjuvant arthritis suppress the disease in arthritis-susceptible rats. Eur. J. Immunol. **25**: 952-957.
31, Macaulay, A.E., R.H. DeKruijff, C.C. Goodnow and D.T. Umetsu. 1997. Antigen-specific B cells preferentially induced CD4⁺ T cells to produce IL-4. J. Immunol. **158**: 4171-4179.
32. Hsieh, C.-S., S.E. Macatonia, C.S. Tripp, S.F. Wolf, A. O'Garra and K.M. Murphy. 1993. Development of T_{H}1 CD4⁺ T cells through IL-12 produced by Listeria-induced macrophages. Science **260**: 547-549.
33. Parronchi, P., M. DeCarli, R. Manetti, C. Simonelli, S. Sampognaro, M.-P. Piccini, D. Macchia, E. Maggi, G. DelPrete and S. Romagnani. 1992. IL-4 and IFN (a and g) exert opposite regulatory effects on the development of cytolytic potential by T_{H}1 or T_{H}2 human T cell clones. J. Immunol. **149**: 2977-2983.
34. Lahdenpohja, N., K. Savinainen and M. Hurme. 1998. Pre-exposure to oxidative stress decreases the nuclear factor - kB-dependent transcription in T lymphocytes. J. Immunol. **160**: 1354-1358.
35. Los, M., W. Droege, K. Stricker, P.A. Bauerle and K. Schulze-Osthoff. 1995. Hydrogen peroxide as a potent activator of T lymphocyte functions. Eur. J. Immunol. **25**: 159-165.
36. Sekiguchi, T. and T. Nagamine. 1994. Inhibition of free radical generation by biotin. Biochem. Pharmacol. **47**: 594-596.
37. Stuehr, D.J. and M.A. Marletta. 1987. Induction of nitrite/nitrate synthesis in murine macrophages by BCG infection, lymphokines or interferon-g. J. Immunol. **139**: 518-525.
38. Huie, R.E. and S. Padmaja. 1993. The reaction of NO with superoxide anion. Free Rad. Res. Commun. **18**: 195-199.
39. Gryglewski, R.J., R.M.J. Palmer and S. Moncada. 1986. Superoxide anion is involved in the breakdown of endothelium-derived vascular relaxing factor. Nature **320**: 454-456.
40. Beckman, J.S., T.W. Beckman, J. Chen, P.A. Marshall and B.A. Freeman. 1990. Apparent hydroxyl radical production by peroxynitrite: implications for endothelial injury from nitric oxide and superoxide. Proc. Natl. Acad. Sci. U.S.A. **87**: 1620-1624.
41. Doherty, D.H., M.P. Doyle, S.R. Curry, R.J. Vali, F.J. Fattor, J.S. Olson and D.D. Lemon. 1998. Rate of reaction with nitric oxide determines the hypertensive effect of cell-free hemoglobin. Nature Biotech. **16**: 672-676.
42. Sandau, K., J. Pfeilschifter and B. Brüne. 1997. The balance between nitric oxide and superoxide determines apoptotic and necrotic death of rat mesangial cells. J. Immunol. **158**: 4938-4946.
43. Zhang, X., T. Brunner, L. Carter, R.W. Dutton, P. Rogers, L. Bradley, T. Sato, J.C. Reed, D. Green and S.L. Swain. 1997. Unequal death in T helper cell (T_{H})1 and T_{H}2 effectors: T_{H}1, but not T_{H}2, effectors undergo rapid Fas/FasL-mediated apoptosis. J. Exp. Med. **185**: 1837-1849.
44. Ding, A.H., C.F. Nathan and D.J. Stuehr. 1988. Release of reactive nitrogen intermediates and reactive oxygen intermediates from mouse peritoneal macrophages. Comparison of activating cytokines and evidence for independent production. J. Immunol. **141**: 2407-2412.
45. Singh, S.P., J.S. Wishnok, M. Keshive, W.M. Deen and S.R. Tannenbaum. 1996. The chemistry of the S-nitrosoglutathione/glutathione system. Proc. Natl. Acad. Sci. U.S.A. **93**: 14428-14433.
46. Rubanyi, G.M., A. Johns, D. Wilcox, F.N. Bates and D. Harrison. 1991. Evidence that a S-nitrosothiol, but not nitric oxide, may be identical with endothelium-derived relaxing factor. J. Cardiovas. Pharmacol. **17**: S41-S45.
47. Hart, T.W. 1985. Some observations concerning the S-nitroso and S-phenyl-sulphonyl derivatives of L-cysteine and glutathione. Tetrahedron Lett. **26**: 2013-2016.
48. Lehn, M., W.Y. Weiser, S. Engelhorn, S. Gillis and H.G. Remold. 1989. IL-4 inhibits H₂O₂ production and antileishmanial capacity of human cultured monocytes mediated by IFN-γ. J. Immunol. **143**: 3020-3024.
49. Brunet, L.R., F.D. Finkelman, A.W. Cheever, M.A. Kopf and E.J. Pearce. 1997. IL-4 protects against TNF-a-mediated cachexia and death during acute Schistosomiasis. J. Immunol. **159**: 777-785.
50. Castedo, M., L. Pelletier, J. Rossert, R. Pasquier, H. Villarroya and P. Druet. 1993. Mercury-induced autoreactive anti-class II T cell line protects from experimental autoimmune encephalomyelitis by the bias of CD8⁺ anti-ergotypic cells in Lewis rats. J. Exp. Med. **177**: 881-889.
51. Pelletier, L., J. Rossert, R. Pasquier, H. Villarroya, M.-F. Belair, M.-C. Vial, R. Oriol and P. Druet. 1988. Effect of HgCl₂ on experimental allergic encephalomyelitis in Lewis rats. HgCl₂-induced down-modulation of the disease. Eur. J. Immunol. **18**: 243-249.

## Claims

1. A method for treating an individual with a condition capable of being influenced by oxygen free radicals comprising administering to said individual at least one compound capable of changing the activity of a pathway for the generation of nitric oxide radicals.

2. A method according to claim 1 wherein the activity of a pathway for the generation of nitric oxide radicals is changed in the vicinity of oxygen free radicals producing cells.

3. A method according to claim 1 or claim 2 wherein the activity of a pathway for the generation of nitric oxide radicals is changed in the vicinity of CD4⁺ T helper cells.

4. A method according to anyone of the claims 1-3, wherein the activity of a pathway for the generation of nitric oxide radicals is changed in the vicinity of cells of the central nervous system.

5. A method according to anyone of the claims 1-4, wherein said pathway is the Rijswijk cycle.

6. A method according to anyone of the claims 1-5, wherein said individual has a condition associated with a malfunctioning immune system.

7. A method according to anyone of the claims 1-6, wherein said compound is capable of decreasing the activity of said pathway.

8. A method according to anyone of the claims 1-7, wherein said compound is a scavenger of peroxynitrite.

9. A method according to anyone of the claims 1-8, wherein said compound is selected from the groups of porphyrin and purine derivatives such as 5,10,15,20-tetrakis (2,4,6-trimethyl-3, 5-disulfonatophenyl) -porphyrinato ironIII(FeTMPS) and uric acid, respectively, and compounds such as deferoxamine (desferrioxamine), ascorbate and Trolex and a combination of two or more of said compounds.

10. A method according to anyone of the claims 1-9, wherein said individual is suffering from a condition associated with a declining number of CD4 cells.

11. A method according to anyone of the claims 1-10, wherein said individual is suffering from a lentivirus infection.

12. A method according to anyone of the claims 1-11, wherein said individual is suffering from a human immunodeficiency virus infection.

13. A method according to anyone of the claims 1-12, wherein said individual is suffering from an allergic disease.

14. A method according to anyone of the claims 1-6, wherein said compound is capable of increasing the activity of said pathway.

15. A method according to anyone of the claims 1-6 or 14, wherein said compound is peroxynitrite, a derivative of peroxynitrite, a peroxynitrite generating compound or a functional analogue of peroxynitrite.

16. A method according to anyone of the claims 1-6, 14 or 15 wherein said compound is used to at least in part decrease a T-helper 1 type immune response in said individual.

17. A method according to anyone of the claims 1-16, wherein said compound is administered in an amount effective for changing the activity of said pathway.

18. A method according to anyone of the claims 1-17, wherein said compound is administered in an amount effective for changing at least partly the immune response in an individual.

19. A method for diagnosing an individual suffering from a condition associated with an increased production of oxygen radicals, comprising providing a sample from said individual and detecting or measuring or both, ammonia and/or nitrite, or derivatives of said compounds in said sample.

20. A peroxynitrite scavenger for use in a method according to anyone of the claims 1-13.

21. A peroxynitrite scavenger according to claim 20, selected from the groups of porphyrin and purine derivatives such as 5,10,15,20-tetrakis(2,4,6-trimethyl-3,5-disulfonatophenyl)-porphyrinato ironIII (FeTMPS) and uric acid, respectively, and compounds such as deferoxamine (desferrioxamine), ascorbate and Trolex.

22. A pharmaceutical composition comprising a peroxynitrite scavenger together with a pharmaceutically acceptable carrier.

23. A pharmaceutical composition according to claim 22, for the treatment of an individual exhibiting a declining number of T helper 1 cells, associated with the presence of oxygen radicals, said treatment comprising administering to said individual at least one compound capable of scavenging peroxynitrite.

24. A pharmaceutical composition according to claim 23 effective for at least reducing the decline of the number of T helper 1 cells.
